# EUROPÄISCHE PATENTSCHRIFT

(11) **EP 4 236 877 B1**
(45) Veröffentlichungstag und Bekanntmachung des Hinweises auf die Patenterteilung: **31.07.2024**
(21) Anmeldenummer: 21798708.0
(22) Anmeldetag: 26.10.2021
(51) Int. Cl.: A61B 17/72, A61F 2/28, A61F 2/30

(54) **PROTHESENANKER**
PROSTHESIS ANCHOR
ANCRAGE DE PROTHÈSE

(30) Priorität: 29.10.2020 DE 102020128512
(43) Veröffentlichungstag der Anmeldung: 06.09.2023
(73) Patentinhaber: Mechamed GmbH, 30938 Burgwedel (DE)
(72) Erfinder: MEIER, Norbert, 30938 Burgwedel (DE)
(74) Vertreter: Scheffler, Jörg
(86) Internationale Anmeldenummer: PCT/EP2021/079675
(87) Internationale Veröffentlichungsnummer: WO 2022/090221

(56) Entgegenhaltungen:
- WO-A2-2007/110863
- DE-U1- 202020 002 112
- US-A- 4 605 350
- US-A- 5 116 378
- US-A1- 2020 129 212

## Beschreibung

Die Erfindung betrifft einen Prothesenanker für eine Prothese, insbesondere eine Endo- oder Exoprothese, mit einer einen Gewindeabschnitt aufweisenden Spannachse zur Übertragung einer axialen Relativbewegung zwischen zumindest einem gegen eine Innenwandfläche einer Ausnehmung in einem Knochen, insbesondere einer Bohrung oder einem Knochenkanal, anlegbaren und in radialer Richtung bezogen auf die Spannachse gegenüber der Innenwandfläche vorspannbaren Hülsenkörper nach dem Oberbegriff des Hauptanspruchs. Weiterhin betrifft die Erfindung eine beispielsweise als Gelenkprothese ausgeführte Prothese mit einem solchen Prothesenanker.

Ein solcher Prothesenanker ist bereits aus der US 5 116 378 A bekannt. Ein proximales Element ist mit einer Bohrung versehen, deren Durchmesser etwas größer als der Durchmesser der Spannachse ist. Bei einer Drehung der Spannachse werden die Keilhülsen nach außen verschoben, wobei zugleich verhindert wird, dass sich die Spannachse seitlich verschiebt.

Aus dem Stand der Technik sind eine Vielzahl von Prothesen mit unterschiedlichen Arten zur Verankerung im Knochen bekannt. Beispielsweise bestehen Gelenkprothesen typischerweise aus einem Prothesenschaft, der im Knochen verankert wird, und einem Gelenkkopf, der mit dem Prothesenschaft verbunden wird und den natürlichen Gelenkkopf ersetzt, wobei unterschiedliche Materialien wie beispielsweise Kunststoff, wie Polyethylen, und Keramik zum Einsatz kommen.

Die bekannten Gelenkprothesen haben überwiegend einen Prothesenschaft mit nagel- oder schraubenförmigen Enden, die in den entsprechenden distalen bzw. proximalen Knochen eingeführt werden.

Nach dem Prinzip der Verankerung der Prothese werden zementfreie und zementierte Prothese unterschieden. Zementfreie Prothesen werden fest in den Knochen getrieben. Eine spezielle raue Oberfläche oder Beschichtung sorgt dafür, dass die Prothese direkt nach dem Einsetzen bereits festsitzt und sich der Knochen dann allmählich mit ihr verbindet. Zementierte Prothesen werden mit einem speziellen Zwei-Komponenten-Kleber befestigt.

Die DE 603 11 269 T2 bezieht sich auf eine Gelenkprothese, bei der mit Hülsen ausgestattete Expansionseinsätze in einem jeweiligen Kanal des Knochens angeordnet werden. Dann wird ein Expansionselement mit einem Innengewinde der Hülse verschraubt. Durch Einschrauben bewegt sich das Expansionselement axial, sodass der konische Schaft des Expansionselements auf die Hülsen einwirkt, die durch Nuten in radialer Richtung elastisch verformbar sind und eine Expansion des kegelstumpfförmigen Außenprofils der Expansionseinsätze verursachen.

Die EP 0 827 385 B1 betrifft einen Nagel zur Lage- und Formfixierung von gebrochenen Röhrenknochen. In einem implantierten Zustand des Nagels wird sein Querschnitt reversibel aufgeweitet. Hierzu wird der Vergrößerungskörper durch Einpumpen eines Gases oder einer Flüssigkeit von innen unter Druck gesetzt und aufgeweitet, wodurch sich ein sternartiger Querschnitt ergibt.

Die DE 103 08 338 A1 bezieht sich auf einen Knochendübel, der in eine Bohrung im Knochen einsteckbar ist und in den eine Schraube eingedreht werden kann, um eine zuverlässige, dauerhafte Verankerung im Knochen zu gewährleisten, beispielsweise zur Fixierung von Knochenfragmenten mittels einer Osteosynthese-Platte. Beim Eindrehen der Schraube erfolgt ein Aufspreizen des Dübels derart, dass die Schraube auf einer Seite des Knochendübels durch einen Schlitz nach außen tritt und am Knochen angreifen kann.

Die US 3 846 846 A beschreibt eine Hüftgelenkkopfprothese mit einem in den Knochenkanal des Oberschenkels eingreifenden und dort festsetzbaren Verankerungsteil mit mehreren Spreizelementen, die mittels eines Spreizelements aufgespreizt werden. Die entstehenden Stufen sorgen zusammen mit der Reibung der Spreizflächen für eine sichere und rutschfeste Verankerung der Prothese. Eine Verdrehsicherung zwischen einer Zugstange und den Spreizelementen wird durch eine Profilierung der Zugstange erreicht.

Als nachteilig erweist es sich bei solchen Fixiermitteln, dass die Verformung durch einen Keilkörper oder durch ein Fluid zwar zu einer vergleichsweise gleichmäßigen Kraftübertragung auf den Knochen führt, allerdings ist die dabei wirkende Vorspannkraft auf den Knochen nur sehr eingeschränkt einstellbar. Zudem kann es bei hohen Belastungen des Prothesenschafts zu einer Kompression des Verformungskörpers kommen, der sich dadurch aus dem Hohlraum lösen kann. Weiterhin kann es bei der Fixierung zu einer unerwünschten Änderung der Orientierung, insbesondere also zu einer Auslenkung aus ihrer zu dem Hohlraum koaxialen Orientierung, kommen, was im Gebrauch, insbesondere bei Gelenkprothesen für den betroffenen Patienten spürbar ist.

Aus der DE 27 03 529 A1 sind Knochennägel bekannt, die in Bohrungen eingebracht werden und infolge des sogenannten Memory-Effekts durch Wärmezufuhr mittels einer Heizung oder durch die Körperwärme aufgeweitet werden. Dabei lassen sich offene Querschnitte mit Wellen- oder Sägezahnprofilen längs des Nagels zur besseren Haftung verwenden.

Aus der DE 32 01 056 C1 ist ein Marknagel bekannt, bei dem der Nagelschaft als Hohlkörper aus einer Memory-Legierung ausgeführt ist, die in Abhängigkeit von der Temperatur jeweils einen von zwei möglichen Formzuständen einnimmt. Damit kann der Marknagel in situ aus einem kleinquerschnittigen in einen aufgeweiteten Zustand überführt werden und umgekehrt.

Nachteilig bei diesem bekannten Marknagel ist die thermische Belastung des Knochens und des Knochenmarks, die die für die Aufweitung des Nagelschafts erforderliche Erwärmung mit sich bringt.

Der Erfindung liegt die Aufgabe zugrunde, einen Prothesenanker zu schaffen, welcher eine definierten Krafteinleitung in radialer Richtung gegenüber der Innenwandfläche und zugleich die Einhaltung einer vorbestimmten, insbesondere zentrierten Orientierung innerhalb der Ausnehmung gestattet. Weiterhin liegt der Erfindung die Aufgabe zugrunde, eine insbesondere als eine Gelenkprothese ausgeführte Prothese mit einem solchen Prothesenanker zu schaffen.

Die erstgenannte Aufgabe wird erfindungsgemäß mit einem Prothesenanker gemäß den Merkmalen des Anspruchs 1 gelöst. Die weitere Ausgestaltung der Erfindung ist den Unteransprüchen zu entnehmen.

Erfindungsgemäß ist also ein Prothesenanker vorgesehen, bei dem die Spannachse mit zumindest einer Keilhülse drehfest verbunden ist. Hierdurch wird erstmals eine Möglichkeit zur lagerichtigen Fixierung des Prothesenankers in dem Hohlraum mittels einer definierten Krafteinleitung erreicht, die zudem ohne Einleitung von Druckfluiden oder thermischer Energie auskommt. Der wesentliche Erfindungsgedanke ist dabei die relative axiale Verlagerung der Keilhülsen, die dabei entlang ihrer Schrägflächen abgleiten und daher entsprechend des Ringspalts mit der Spannachse radial ausgelenkt werden. Indem die Spannachse zumindest mit der distalen Keilhülse drehfest verbunden und die Spannachse mit einer Werkzeugaufnahme ausgestattet ist, um die Spannachse und somit die Keilhülse während der Fixierung drehfest zu arretieren, bleibt nicht nur die gewünschte Orientierung der Spannachse unverändert erhalten, sondern es wirkt auch kein Drehmoment zwischen den Keilhülsen und der Innenwandfläche, sodass insbesondere unerwünschte Scherkräfte vermieden werden. Von dem mittels des beispielsweise als Hülsenmutter ausgeführten Spannkörpers eingeleiteten Drehmoment zur relativen axialen Verlagerung der Keilhülsen kann zudem auf der Basis von Erfahrungswerten mit ausreichender Genauigkeit auf die radiale Krafteinwirkung der Keilhülsen gegenüber der Innenwandfläche geschlossen werden, sodass hier durch eine Drehmomentbegrenzung eine Überlastung vermieden und erforderliche Haltekräfte problemlos erreicht werden. Selbstverständlich kann das eingeleitete Drehmoment auch messtechnisch überwacht und protokolliert bzw. einer Datenverarbeitung zugeführt werden. Durch das Abgleiten der Keilhülsen entlang ihrer Schrägflächen kommt es zudem zu einer gleichmäßigen, synchronen radialen Verlagerung der Keilhülsen in radialer Richtung, ohne dass hiermit eine unerwünschte Verlagerung bzw. Auslenkung der Spannachse verbunden ist. Im Gegensatz zum Stand der Technik ändert sich somit die Position und Orientierung der Spannachse während der Fixierung nicht, sodass enge Toleranzen bei der Positionierung eingehalten werden können und dadurch neben der Haltbarkeit auch die Funktionsfähigkeit der auf diese Weise fixierten Prothese wesentlich verbessert werden kann. Dabei hat sich bereits gezeigt, dass die Keilhülsen erfindungsgemäß gegenüber der Spannachse parallel verlagert werden und dadurch flächig gegen die Innenwandfläche anliegen. Dadurch wird eine optimale Kraftübertragung erreicht, die durch eine geeignete Oberflächenbeschaffenheit der Keilhülsen, beispielsweise eine Strukturierung oder Profilierung, noch weiter verbessert werden kann.

Die Keilhülsen könnten beispielsweise eine polygonale Querschnittsform aufweisen. Als vorteilhaft hat es sich demgegenüber bereits erwiesen, wenn die Keilhülsen eine rotationssymmetrische, insbesondere zylindrische Grundform aufweisen.

Dabei bestehen die Keilhülsen bevorzugt aus einem hochwertigen, für Medizinprodukte geeigneten Metall, wobei Kunststoffe nicht grundsätzlich ausgeschlossen sind. Beispielsweise sind Keilhülsen aus GFK oder CFK realisierbar, die gegebenenfalls mit einem metallischen Lageindikator versehen sein können, um die Gebrauchsposition durch Röntgenverfahren überprüfen zu können.

Die Keilhülsen können entlang ihrer Schrägflächen unmittelbar gegeneinander anliegen. Eine besonders praxisnahe Ausgestaltungsform der Erfindung wird hingegen dadurch erreicht, dass der Prothesenanker zumindest ein zwischen den Keilhülsen angeordnetes, gegen die Schrägflächen gegenüberliegender Keilhülsen anliegendes Führungsteil aufweist, welches aufgrund seiner Materiabeschaffenheit, beispielsweise aus Kunststoff, dazu dient, die Reibungskräfte bei der radialen Verlagerung der Keilhülsen zu reduzieren und insbesondere einen unerwünschten Materialabrieb, der anderenfalls in den Knochen eindringen könnte, zu vermeiden. Darüber hinaus eignet sich das Führungsteil als Abdeckelement zum Verschließen der Spalte bzw. Schlitze der Keilhülsen ebenso wie sonstiger Ausnehmungen an der Spannachse, sodass die Oberfläche frei von Hinterschneidungen ist. Das Führungsteil kann aber auch im Hinblick auf das Einwachsen in den Knochen optimierte Eigenschaften aufweisen. Weiterhin kann das Führungsteil zur einfachen Längenanpassung ausgetauscht werden.

Besonders bevorzugt ist zumindest der durch die Keilhülsen begrenzte Abschnitt des Prothesenankers durch einen flexiblen, beispielsweise schlauchförmigen, Hüllkörper eingeschlossen, um so eine für das Knochengewebe günstige Zwischenschicht in den Hohlraum einzubringen. Zudem werden dadurch die Bestandteile des Prothesenankers auch in der Nichtgebrauchsstellung von dem Hüllkörper unverlierbar eingeschlossen.

Eine weitere, ebenfalls besonders vorteilhafte Ausführungsform der Erfindung wird auch dadurch realisiert, dass der Spannkörper in der Gebrauchsposition relativ zu der Spannachse mittels einer Drehsicherung drehfest fixiert ist. Der beispielsweise als Spannknebel zur manuellen Betätigung ausgeführte Spannkörper dient dazu, die axiale Vorspannkraft auf die Keilhülsen zu übertragen. In der Gebrauchsposition wird dieser drehfest fixiert, indem die Drehsicherung zugleich mittels einer formschlüssigen Ausnehmung den nicht-rotationssymmetrischen Spannkörper einschließt und mit einem Vorsprung in einer Ausnehmung der Spannachse eingreift. Dadurch ist die Drehsicherung mit der Spannachse drehfest verbunden. Die Drehsicherung wird ihrerseits durch ein in axialer Richtung wirkendes Fixiermittel, beispielsweise eine Schraube, gesichert. Hierzu weist die Spannachse in dem Endabschnitt zusätzlich zu dem Außengewinde für den Spannknebel ein Innengewinde für das Fixiermittel auf.

Die Spannachse könnte unlösbar oder einteilig mit einem Funktionsträger der Prothese, beispielsweise einem Gelenkkopf, verbunden sein. Besonders praxisgerecht ist hingegen eine Variante der Erfindung, bei welcher die Spannachse eine Ausformung für eine Werkzeugaufnahme und/oder ein Funktionselement der Prothese aufweist, sodass das Funktionselement im Anschluss an die Fixierung der Spannachse mit dieser verbunden und entsprechend ausgerichtet oder justiert werden kann. Die Spannachse ermöglicht dadurch einen universellen Einsatz und ist dabei insbesondere nicht auf Prothesen im engeren Sinne beschränkt, sondern kann beispielsweise auch zur Fixierung von Knochenplatten oder sonstigen, nach einer Heilung von dem Körper zu trennenden medizinischen Hilfsmitteln dienen.

Weiterhin hat es sich bereits als besonders zweckmäßig erwiesen, wenn die Spannachse eine die Ausformung aufweisende, radial erweiterte Druckplatte zur insbesondere formschlüssigen Aufnahme der Drehsicherung aufweist. Die Druckplatte dient dabei als Abstützung des Prothesenankers an einem die Ausnehmung in dem Knochen umgebenden Randbereich und somit zugleich als Tiefenanschlag für eine definierte Positionierung des Prothesenankers in dem Knochen. Die Druckplatte hat vorzugsweise eine rotationssymmetrische Grundform und eine ebene Stützfläche als Widerlager für den Spannkörper.

Eine andere, ebenfalls besonders vorteilhafte Ausgestaltungsform der Erfindung wird dadurch realisiert, dass zumindest eine der Keilhülsen, insbesondere in Längsrichtung durchgehend geschlitzt ausgeführt ist, wobei in der Gebrauchsposition zumindest ein radialer Vorsprung der Spannachse in den Schlitz eingreift und die Keilhülse drehfest mit der Spannachse verbindet. Indem jeweils zumindest ein Vorsprung der Spannachse in jeweils einen Schlitz der beiden Keilhülsen eingreift, sind diese relativ zu der Spannachse drehfest mit dieser verbunden. Somit kommt es bei dem axialen Verspannen der Keilhülsen und der damit verbundenen radialen Verlagerung nicht zu einer unerwünschten Drehbewegung der Keilhülsen.

Bei einer bevorzugten Variante weist zumindest eine der Keilhülsen einen in Längsrichtung durchgehenden Schlitz mit einer Schlitzbreite größer als der Durchmesser der Spannachse in der Querschnittsebene auf, sodass die Keilhülsen quer zu der Spannachse auf diese aufgesetzt und problemlos ausgetauscht werden können.

Die zweitgenannte Aufgabe, eine insbesondere als eine Gelenkprothese ausgeführte Prothese mit einem solchen Prothesenanker zu schaffen, wird erfindungsgemäß dadurch gelöst, dass der Hülsenkörper zumindest zwei relativ zueinander bewegliche Keilhülsen aufweist, die entlang einer gegenüber der Querschnittsebene zu ihrer Längsachse geneigten Schrägfläche als Kontaktfläche gegeneinander oder gegen ein zwischen den Keilhülsen eingeschlossenes Führungsteil anliegen und aufgrund einer relativen axialen Verlagerung und Annäherung zugleich auch radial verlagert werden, soweit dies der Ringspalt zwischen dem Innendurchmesser der Keilhülsen und der eingeschlossenen Spannachse zulässt. Die als formstabile Hohlkörper ausgeführten Keilhülsen können dadurch mit einer definierten Vorspannkraft gegen die Innenwandfläche des Knochenhohlraums angepresst werden.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: eine perspektivische Darstellung eines erfindungsgemäßen Prothesenankers;
- Fig. 2: eine Gebrauchsposition des in einen Knochenhohlraum eingesetzten Prothesenankers in einer geschnittenen Seitenansicht;
- Fig. 3: eine perspektivische Darstellung des Prothesenankers mit gelöstem Spannkörper;
- Fig. 4: eine perspektivische Darstellung des Prothesenankers mit fixiertem Spannkörper;
- Fig. 5: eine perspektivische Darstellung des Prothesenankers mit dem durch eine Drehsicherung festgelegten Spannkörper.

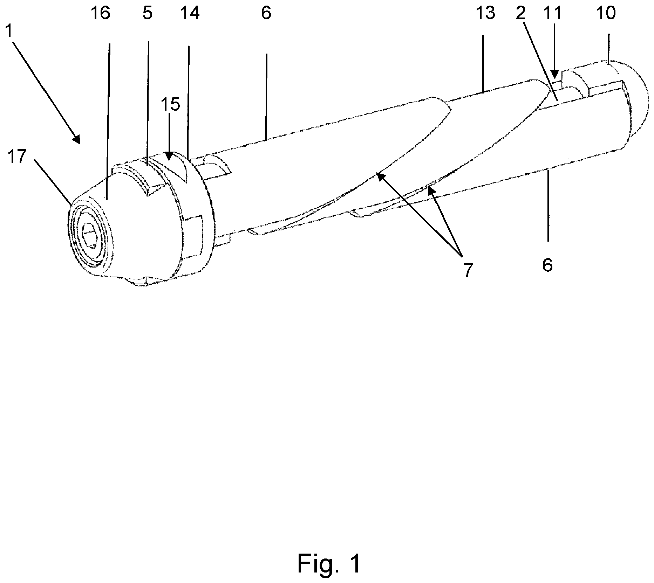

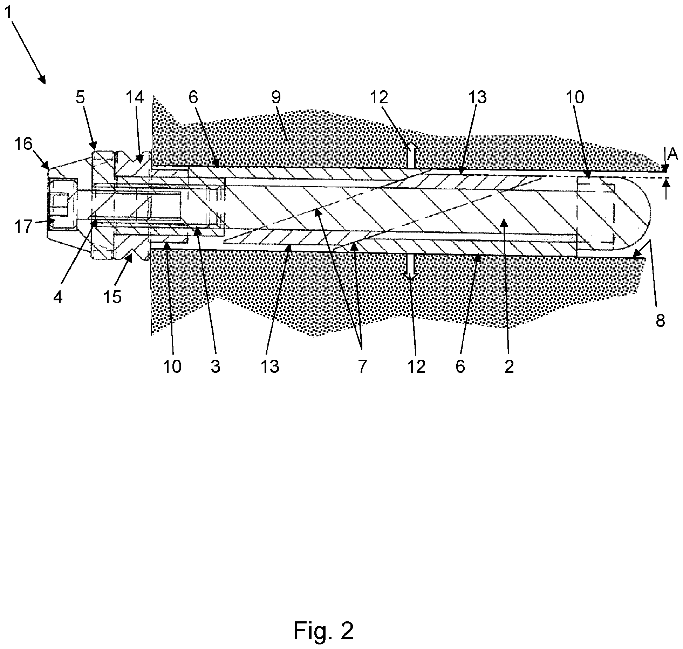

Ein erfindungsgemäßer Prothesenanker 1 für eine nicht weiter dargestellte Prothese wird nachstehend anhand der Figuren 1 bis 5 näher erläutert. Der Prothesenanker 1 hat eine Spannachse 2 mit einem Gewindeabschnitt 3, auf den ein mit einem Innengewinde 4 ausgestatteter Spannkörper 5 aufschraubbar ist. Durch die dabei entstehende axiale Relativbewegung werden zwei Keilhülsen 6, die mit einer gegenüber der Querschnittsebene der Spannachse 2 geneigten Schrägfläche 7 ausgestattet sind, radial verschoben, bis diese schließlich gegen eine Innenwandfläche 8 eines in Figur 2 andeutungsweise dargestellten Knochenhohlraums eines Knochens 9 anliegen.

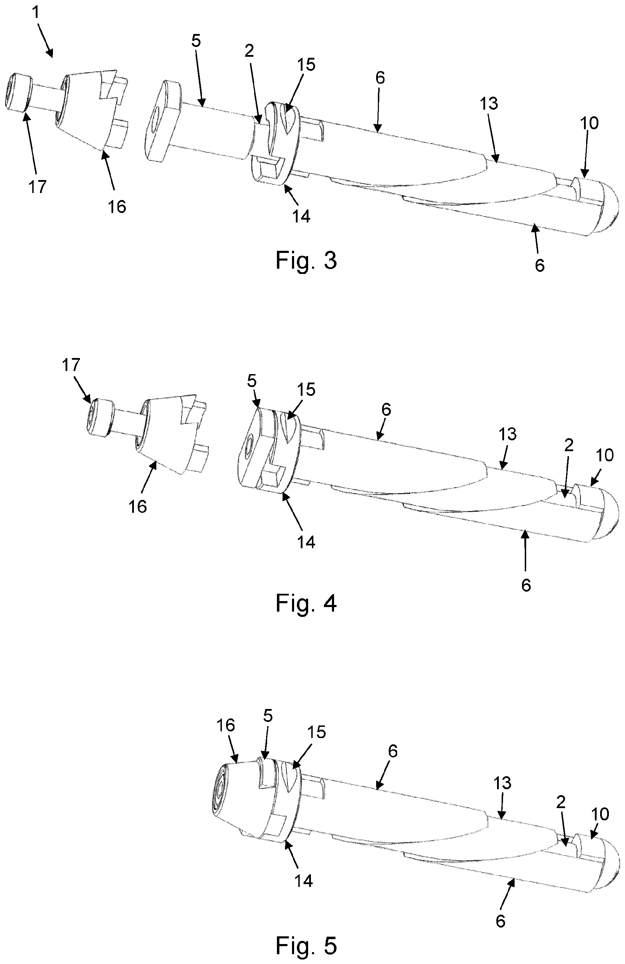

Die einzelnen Arbeitsschritte bei der Fixierung des Prothesenankers 1 werden anhand der Figuren 3 bis 5 weiter verdeutlicht. Die Spannachse 2 bildet ein Grundelement und trägt die beiden geschlitzten Keilhülsen 6. Dabei greift jeweils ein radialer Vorsprung 10 der Spannachse 2 in einen jeweiligen Schlitz 11 der Keilhülsen 6 ein, sodass diese mit der Spannachse 2 drehfest verbunden sind. Mittels des als Spannknebel ausgeführten Spannkörpers 5 lassen sich die Keilhülsen 6 manuell verspannen. Indem der lichte Innendurchmesser der Keilhülsen 6 geringfügig größer als der Durchmesser der Spannachse 2 bemessen ist, wird eine relative radiale Verlagerung gegenüber der Spannachse 2 in entgegengesetzten Richtungen in Pfeilrichtung 12 quer zu der Spannachse 2 mit einem Abstand A ermöglicht. Insbesondere werden durch Drehen des Spannkörpers 5 die Keilhülsen 6 mittels eines eingeschlossenen Führungsteils 13 gegeneinander verspannt und gleiten an dessen korrespondierenden Flächen ab. Dadurch verspannen sich die Keilhülsen 6 in dem Hohlraum des Knochens 9 an gegenüberliegenden Flächenabschnitten der Innenwandfläche 8. Das zwischen den Keilhülsen 6 angeordnete Führungsteil 13 dient dabei der Zentrierung des Prothesenankers 1 in dem Hohlraum des Knochens 9.

Wie in Figur 4 zu erkennen ist, liegt in der vorgespannten Gebrauchsposition der Keilhülsen 6 der Spannkörper 5 gegen eine als Flansch oder Kragen ausgeführte Stirnfläche einer Druckplatte 14 axial an. Die Druckplatte 14 dient zugleich als Basis zur insbesondere form- und/oder kraftschlüssigen Fixierung von Funktionselementen der Prothese mittels einer Ausformung 15 für eine Werkzeugaufnahme und ein Funktionselement der Prothese.

Gesichert wird die so eingestellte Gebrauchsposition durch eine Verdrehsicherung 16, welche die Druckplatte 14 und den Spannkörper 5 in der Gebrauchsposition drehfest verbindet und so ein unbeabsichtigtes Lösen der Verbindung verhindert, wie in Figur 5 gezeigt. Die Verdrehsicherung 16 wird ihrerseits durch ein in axialer Richtung wirkendes, als Schraube ausgeführtes Fixiermittel 17 gesichert, indem das Fixiermittel 17 in das Innengewinde 4 in dem Endabschnitt der Spannachse 2 eingeschraubt wird.

### BEZUGSZEICHENLISTE

| | | | |
|---|---|---|---|
| 1 | Prothesenanker | 16 | Verdrehsicherung |
| 2 | Spannachse | 17 | Fixiermittel |
| 3 | Gewindeabschnitt | | |
| 4 | Innengewinde | A | Abstand |
| 5 | Spannkörper | | |
| 6 | Keilhülse | | |
| 7 | Schrägfläche | | |
| 8 | Innenwandfläche | | |
| 9 | Knochen | | |
| 10 | Vorsprung | | |
| 11 | Schlitz | | |
| 12 | Pfeilrichtung | | |
| 13 | Führungsteil | | |
| 14 | Druckplatte | | |
| 15 | Ausformung | | |

## Patentansprüche

1. Prothesenanker (1) für eine Prothese mit einer einen Gewindeabschnitt (3) aufweisenden Spannachse (2) zur Übertragung einer axialen Relativbewegung zwischen zumindest einem gegen eine Innenwandfläche (8) einer Ausnehmung in einem Knochen (9) anlegbaren und in radialer Richtung bezogen auf die Spannachse (2) gegenüber der Innenwandfläche (8) vorspannbaren Hülsenkörper, wobei der Hülsenkörper zumindest zwei relativ zueinander bewegliche Keilhülsen (6) aufweist, die jeweils eine gegenüber der Querschnittsebene zu ihrer Längsachse geneigten Schrägfläche (7) aufweisen, wobei der Innendurchmesser der Keilhülsen (6) größer ist als der Durchmesser der Spannachse (2), sodass jede Keilhülse (6) in der Nichtgebrauchsposition mit der Spannachse (2) einen Ringspalt einschließt und die Keilhülsen (6) in der Gebrauchsposition mittels eines eine Gewindeaufnahme für den Gewindeabschnitt (3) der Spannachse (2) aufweisenden Spannkörpers (5) axial gegeneinander derart verspannt sind, dass die Keilhülsen (6) in der Gebrauchsposition mit einem Abstand (A) aus der Nichtgebrauchsposition in entgegengesetzten Richtungen quer zu der Spannachse (2) verlagert sind, **dadurch gekennzeichnet, dass** die Spannachse (2) mit zumindest einer Keilhülse (6) drehfest verbunden ist.

2. Prothesenanker (1) nach Anspruch 1, **dadurch gekennzeichnet, dass** der Prothesenanker (1) zumindest ein zwischen den Keilhülsen (6) angeordnetes, gegen die Schrägflächen (7) gegenüberliegender Keilhülsen (6) anliegendes Führungsteil (13) aufweist.

3. Prothesenanker (1) nach Anspruch 1 oder 2, **dadurch gekennzeichnet, dass** der Spannkörper (5) in der Gebrauchsposition relativ zu der Spannachse (2) mittels einer Verdrehsicherung (16) drehfest fixiert ist.

4. Prothesenanker (1) nach einem der vorhergehenden Ansprüche, **dadurch gekennzeichnet, dass** die Spannachse (2) eine Ausformung (15) für eine Werkzeugaufnahme und/oder ein Funktionselement der Prothese aufweist.

5. Prothesenanker (1) nach Anspruch 4, **dadurch gekennzeichnet, dass** die Spannachse (2) eine die Ausformung (15) aufweisende, radial erweiterte Druckplatte (14) aufweist.

6. Prothesenanker (1) nach zumindest einem der vorhergehenden Ansprüche, **dadurch gekennzeichnet, dass** zumindest eine der Keilhülsen (6), insbesondere in Längsrichtung, durchgehend geschlitzt ausgeführt ist, wobei in der Gebrauchsposition zumindest ein radialer Vorsprung (10) der Spannachse (2) in den Schlitz (11) eingreift und die Keilhülse (6) drehfest mit der Spannachse (2) verbindet.

7. Prothesenanker (1) nach zumindest einem der vorhergehenden Ansprüche, **dadurch gekennzeichnet, dass** zumindest eine der Keilhülsen (6) einen in Längsrichtung durchgehenden Schlitz (11) mit einer Schlitzbreite größer als der Durchmesser der Spannachse (2) in der Querschnittsebene aufweist.

8. Prothesenanker (1) nach zumindest einem der vorhergehenden Ansprüche, **dadurch gekennzeichnet, dass** zumindest der durch die Keilhülsen (6) begrenzte Abschnitt des Prothesenankers (1) durch einen flexiblen, beispielsweise schlauchförmigen, Hüllkörper eingeschlossen ist.

9. Prothesenanker (1) nach zumindest einem der vorhergehenden Ansprüche, **dadurch gekennzeichnet, dass** zumindest eine Keilhülse (6) eine polygonale oder eine rotationssymmetrische, insbesondere zylindrische, Querschnittsform aufweist.

10. Eine mit einem Prothesenanker (1) nach zumindest einem der vorhergehenden Ansprüche ausgestattete Prothese.

## Claims

1. Prosthesis anchor (1) for a prosthesis, with a clamping shaft (2) having a threaded portion (3) for transmitting an axial relative movement between at least one sleeve body which can be placed against an inner wall surface (8) of a recess in a bone (9) and can be pretensioned, in a radial direction with respect to the clamping shaft (2), against the inner wall surface (8), wherein the sleeve body has at least two wedge sleeves (6) which are movable relative to each other and which each have an oblique surface (7) which is inclined relative to the cross-sectional plane to their longitudinal axis, wherein the internal diameter of the wedge sleeves (6) is greater than the diameter of the clamping sleeve (2), such that each wedge sleeve (6) encloses an annular gap with the clamping shaft (2) in the non-use position, and the wedge sleeves (6), in the use position, are braced axially against each other, by means of a clamping body (5) having a threaded receptacle for the threaded portion (3) of the clamping shaft (2), in such a way that the wedge sleeves (6) in the use position are displaced by a distance (A) from the non-use position in opposite directions transverse to the clamping shaft (2), **characterized in that** the clamping shaft (2) is connected to at least one wedge sleeve (6) for conjoint rotation.

2. Prosthesis anchor (1) according to Claim 1, **characterized in that** the prosthesis anchor (1) has at least one guide part (13) arranged between the wedge sleeves (6) and bearing against the oblique surfaces (7) of opposite wedge sleeves (6).

3. Prosthesis anchor (1) according to Claim 1 or 2, **characterized in that** the clamping body (5) in the use position is rotationally fixed relative to the clamping shaft (2) by means of an anti-rotation device (16).

4. Prosthesis anchor (1) according to any one of the preceding claims, **characterized in that** the clamping shaft (2) has a formation (15) for a tool holder and/or a functional element of the prosthesis.

5. Prosthesis anchor (1) according to Claim 4, **characterized in that** the clamping shaft (2) has a radially extended pressure plate (14) having the formation (15).

6. Prosthesis anchor (1) according to at least one of the preceding claims, **characterized in that** at least one of the wedge sleeves (6) is continuously slotted, in particular in the longitudinal direction, wherein at least one radial projection (10) of the clamping shaft (2) engages in the slot (11) in the use position and connects the wedge sleeve (6) to the clamping shaft (2) for conjoint rotation.

7. Prosthesis anchor (1) according to at least one of the preceding claims, **characterized in that** at least one of the wedge sleeves (6) has a continuous slot (11) in the longitudinal direction, with a slot width greater than the diameter of the clamping shaft (2) in the cross-sectional plane.

8. Prosthesis anchor (1) according to at least one of the preceding claims, **characterized in that** at least the portion of the prosthesis anchor (1) delimited by the wedge sleeves (6) is enclosed by a flexible, for example tubular, enveloping body.

9. Prosthesis anchor (1) according to at least one of the preceding claims, **characterized in that** at least one wedge sleeve (6) has a polygonal or a rotationally symmetrical, in particular cylindrical, cross-sectional shape.

10. Prosthesis equipped with a prosthesis anchor (1) according to at least one of the preceding claims.

## Revendications

1. Ancrage de prothèse (1) destiné à une prothèse et comportant un axe de serrage (2) pourvu d'une portion filetée (3) pour transmettre un mouvement relatif axial entre au moins un corps de manchon qui peut être placé dans un os (9) contre une surface de paroi intérieure (8) d'un évidement et qui peut être précontraint contre la surface de paroi intérieure (8) dans une direction radiale par rapport à l'axe de serrage (2), le corps de manchon comportant au moins deux manchons formant cale (6) qui sont mobiles l'un par rapport à l'autre et qui comportent chacun une surface oblique (7) qui est inclinée par rapport au plan en coupe transversale par rapport à son axe longitudinal, le diamètre intérieur des manchons formant cale (6) étant supérieur au diamètre de l'axe de serrage (2) de sorte que chaque manchon formant cale (6), dans la position de nonutilisation, forme avec l'axe de serrage (2) un espace annulaire et les manchons formant cale (6), dans la position d'utilisation, étant serrés axialement les uns contre les autres au moyen d'un corps de serrage (5) qui comporte un logement fileté destiné à la portion filetée (3) de l'axe de serrage (2) de manière à ce que les manchons formant cale (6), dans la position d'utilisation, soient déplacés dans des directions opposées transversalement à l'axe de serrage (2) à une distance (A) par rapport à la position de nonutilisation, **caractérisé en ce que** l'axe de serrage (2) est relié solidairement en rotation à au moins un manchon formant cale (6).

2. Ancrage de prothèse (1) selon la revendication 1, **caractérisé en ce que** l'ancrage de prothèse (1) comporte au moins une partie de guidage (13) qui est disposée entre les manchons formant cale (6) et qui vient en appui contre les surfaces obliques (7) de manchons formant cale (6) opposés.

3. Ancrage de prothèse (1) selon la revendication 1 ou 2, **caractérisé en ce que** le corps de serrage (5) est fixé solidairement en rotation dans la position d'utilisation par rapport à l'axe de serrage (2) au moyen d'une sécurité anti-rotation (16).

4. Ancrage de prothèse (1) selon l'une des revendications précédentes, **caractérisé en ce que** l'axe de serrage (2) comporte une partie saillante (15) destinée à un porte-outil et/ou un élément fonctionnel de la prothèse.

5. Ancrage de prothèse (1) selon la revendication 4, **caractérisé en ce que** l'axe de serrage (2) comporte une plaque de pression (14) qui est élargie radialement et qui comporte la partie saillante (15).

6. Ancrage de prothèse (1) selon l'une au moins des revendications précédentes, **caractérisé en ce qu'**au moins un des manchons formant cale (6) est pourvu d'une fente continue, notamment dans la direction longitudinale, au moins une saillie radiale (10) de l'axe de serrage (2) s'engageant, dans la position d'utilisation, dans la fente (11) et reliant solidairement en rotation le manchon formant cale (6) à l'axe de serrage (2) .

7. Ancrage de prothèse (1) selon l'une au moins des revendications précédentes, **caractérisé en ce qu'**au moins un des manchons formant cale (6) comporte une fente continue (11) dans la direction longitudinale dont la largeur est supérieure au diamètre de l'axe de serrage (2) dans le plan en coupe transversale.

8. Ancrage de prothèse (1) selon l'une au moins des revendications précédentes, **caractérisé en ce qu'**au moins la portion de l'ancrage de prothèse (1) qui est délimitée par les manchons formant cale (6) est formée par un corps d'enveloppe souple, par exemple en forme de tuyau.

9. Ancrage de prothèse (1) selon l'une au moins des revendications précédentes, **caractérisé en ce qu'**au moins un manchon formant cale (6) a une forme de coupe transversale polygonale ou à symétrie de rotation, notamment cylindrique.

10. Prothèse équipée d'un ancrage de prothèse (1) selon l'une au moins des revendications précédentes.
